## Europäisches Patentamt

## European Patent Office

## Office européen des brevets

(11) Numéro de publication: **0 135 441**

**B1**

---

(12) # FASCICULE DE BREVET EUROPÉEN

(45) Date de publication du fascicule du brevet:
**05.11.86**

(21) Numéro de dépôt: **84401784.8**

(22) Date de dépôt: **11.09.84**

(51) Int. Cl.⁴: **C 07 C 11/08,** C 07 C 2/30,
B 01 J 31/14

---

(54) **Procédé amélioré de synthèse du butène-1 par dimérisation de l'éthylène.**

---

(30) Priorité: **20.09.83 FR 8315040**
**20.09.83 FR 8315041**

(43) Date de publication de la demande:
**27.03.85 Bulletin 85/13**

(45) Mention de la délivrance du brevet:
**05.11.86 Bulletin 86/45**

(84) Etats contractants désignés:
**BE DE GB IT NL SE**

(56) Documents cité:
**FR-A-2 341 540**
**GB-A-1 235 631**

(73) Titulaire: **INSTITUT FRANCAIS DU PETROLE, 4, Avenue de Bois- Préau, F-92502 Rueil- Malmaison (FR)**

(72) Inventeur: **Le Quan, Nhuong, 20, Allée des Acacias, F-78410 Aubergenville (FR)**
Inventeur: **Cruypelinck, Daniel, 16, Rue Claude Tillet "Ognes", F-69440 Nanteuil- Le- Haudouin (FR)**
Inventeur: **Commereuc, Dominique, 32, Rue Abel Vacher, F-92190 Meudon (FR)**
Inventeur: **Chauvin, Yves, 64, Av. du Général Leclerc, F-78230 Le Pecq (FR)**
Inventeur: **Leger, Gérard, 2, Av. de Veyssières Résidence du Parc Allée 14, F-69130 Ecully (FR)**

---

EP 0 135 441 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

L'objet de la présente invention est un procédé amélioré de dimérisation de l'éthylène en butène-.

Dans le brevet US 2 943 125, K. Ziegler a décrit une méthode de dimérisation de l'éthyléne en butène-1 au moyen d'un catalyseur obtenu par le mélange de trialkylaluminium et d'un tétraalcoolate de titane ou de zirconium lors de la réaction se forme également une certaine quantité de polyéthylène de haute masse moléculaire qui gêne considérablement la mise en oeuvre. Plusieurs améliorations ont été proposées pour diminuer le taux de polymère, en particulier dans le brevet US 3 686 350 qui préconise l'emploi de composés organiques du phosphore, conjointement avec les éléments du catalyseur, dans le brevet US 4 101 600 qui revendique le traitement du catalyseur par de l'hydrogène ou dans le brevet US 3 579 455 qui revendique l'utilisation de divers éthers comme solvants du milieu réactionnel. Bien que ces modifications du système catalytique initial apportent une amélioration substantielle à la sélectivité de la réaction, elles se révèlent d'une utilisation peu pratique, en particulier dans un procédé industriel dans lequel il faut pouvoir séparer le butène-1 du solvant en laissant seulement des traces de composés polaires dans le butène.

L'objet de la présente invention est de décrire un catalyseur présentant une activité et une sélectivité exceptionnelles. Cet effet est plus marqué que celui qui est apporté par les coordinats revendiqués jusqu'à présent tels que les phosphites ou les amines, et il est également plus marqué que celui apporté par les éthers quand ces derniers sont utilisés en quantité correspondant à un usage comme solvant ou quand ils sont ajoutés après mélange de l'hydrocarbylaluminium avec le titanate d'alkyle. Le procédé présente également l'avantage d'éliminer l'emploi desdits éthers comme solvants, dont on a signalé les inconvénients (difficultés de séparation). Le procédé permet même d'éviter l'usage d'un solvant d'origine externe, les constituants du catalyseur pouvant être mélangés directement au sein d'un des produits ou des sous produits de la réaction, ce qui évite la consomnation ou le recyclage dudit solvant, toujours difficile à séparer d'un mélange issu d'une oligomérisation.

L'invention concerne ainsi un procédé amélioré de conversion de l'éthylène en butène-1, dans lequel on met l'éthylène en contact avec un catalyseur obtenu par intéraction d'un titanate d'alkyle avec un conposé d'alkylaluminium, caractérisé en ce que ledit catalyseur résulte de l'intéraction d'un mélange préformé de titanate d'akyle et d'éther dans un rapport molaire éther/titanate de 0,5: 1 à 10: 1 avec un composé d'aluminium de formule $AlR_3$ ou $AlR_2H$ dans laquelle chacun des restes R est un radical hydrocarbyle.

Les éthers sont ainsi utilisés dans un rapport molaire de 0,5 à 10 de préférence 1 à 3, plus particulièrement 2 moles d'éther par mole de composé du titane. Sans être lié à aucune théorie, on peut penser que l'éther se complexe sur l'atome de titane permettant l'hexacoordination que le titane ne réalise autrement qu'en s'auto associant. Si on met en oeuvre l'éther dans des rapports supérieurs à 10, tels que 20 et au-delà, ou si il est utilisé comme solvant de réaction on observe que la réaction est considérablement ralentie et que se sélectivité est moins bonne, et même, dans certains cas, la réaction ne se produit pas du tout.

Les composés de l'aluminium utilisés pour préparer le catalyseur sont représentés par les formules générales $AlR_3$ ou $AlR_2H$ dans lesquelles R est un radical hydrocarbyl, de préférence alkyle comprenant de 2 à 6 atomes de carbone, par exemple le triéthylaluminium, le tripropylaluminium, le triisobutylaluminium, l'hydrure de diisobutylaluminium, le trihexylaluminium.

Les éthers utilisés dans l'invention peuvent être des monoéthers ou des polyéthers. On peut utiliser par exemple le diéthyléther, le diisopropyléther, le dibutyléther, le méthyltertiobutyléther, le tétrahydrofuranne, le dioxanne-1,4, le dihydropyranne, l'éther diméthylique de l'éthylène glycol.

Des résultats exceptionnels sont obtenus avec le tétrahydrofuranne et/ou le dioxanne-1,4.

Les titanates d'alkyle utilisés dans l'invention répondent à la formule générale $Ti(OR')_4$ dans laquelle R' est un radical alkyle linéaire ou ramifié comportant de préférence de 2 à 8 atomes de carbone. On peut utiliser par exemple le titanate de tétraéthyle, le titanate de tétraisopropyle, le titanate de tétra-n-butyle, le titanate de tétra-2-éthyl-hexyle.

Les composants du catalyseur peuvent être mis en contact au sein d'un hydrocarbure et/ou d'un ou des produits d'oligomérisation tels que les hexènes, de préférence en présence d'éthylène. Le rapport molaire entre le composé de l'aluminium et celui du titane est d'environ 1: 1 à 20: 1 et de préférence de environ 2: 1 à 5: 1. La concentration du titane dans la solution ainsi préparée est avantageusement comprise entre $10^{-4}$ et 0,5 mole par l'itre et de préférence entre $2.10^{-3}$ et 0,1 mole par litre. La température à laquelle se fait la préparation du catalyseur est habituellement comprise entre -10 et + 80°C, de préférence entre -10 et + 45°C et de manière encore plus préférée entre 0 et + 40°C. Quand de l'éthylène est présent dans le milieu, sa quantité correspond de préférence à la saturation de la solution à la température considérée et à la pression choisie d'une atmosphère ou davantage.

La solution de catalyseur ainsi obtenue peut être utilisée telle quelle ou elle peut être diluée par addition des produits de la réaction.

La réaction de dimérisation de l'éthylène peut être mise en oeuvre à 20-150°C, de prérérence 20-70°C et de manière encore plus préférée 50-70°C.

La pression est de préférence de 0,5 à 8 MPa.

Dans un mode particulier de mise en oeuvre de la réaction catalytique de dimérisation en discontinu, on introduit un volume choisi de la solution catalytique, préparée comme décrit ci-dessus, dans un réacteur muni des habituels systèmes d'agitation et de refroidissement, puis on pressurise par de l'éthylène à une pression de préférence comprise entre 0,5 et 8 MPa et on maintient la température entre 20 et 70°C, de préférence entre 50 et 70°C. On alimente le réacteur par de l'éthylène à pression constante jusqu'à ce que le volume total de liquide produit représente entre 2 et 50 fois le volume de la solution catalytique primitivement introduit; on deétruit alors le catalyseur, par exemple par addition d'eau, et on soutire et sépare les produits de la réaction et le solvant éventuel.

En cas d'opération continue, la mise en oeuvre est de préférence la suivante: la solution catalytique est injectée en même temps que l'éthylène dans un réacteur agité par les moyens classiques ou par recirculation extérieure. La température est maintenue entre environ 20° et 70°C, de préférence entre 50 et 70°C, et la pression est de préférence comprise entre 0,5 et 8 MPa. Par une vanne de détente qui maintient la pression constante, s'écoule une partie du mélange réactionnel à un débit massique égal au débit massique des fluides introduits. Le fluide ainsi détendu est envoyé dans un système de colonnes à distiller qui permet de séparer le butène-1 de l'éthylène d'une part, qui est renvoyé au réacteur, des hexènes et des octènes d'autre part, dont une partie est renvoyée dans la section de préparation du catalyseur. Le pied de colonne contenant le catalyseur et les produits lourds peut être incinéré, ou bien le catalyseur récupéré est recyclé.

Dans un mode de réalisation préféré, on mélange les composants du catalyseur en atmosphère d'éthylène à une température de -10 à + 45°C, puis on augmente la température jusqu'à 50-70°C et on laisse la réaction de dimérisation se poursuivre. De manière encore plus préférée, on maintient les composants mélangés du catalyseur à une température de -10 à + 45°C pendant 30 secondes à 24 heures avant de porter ensuite la température jusqu'à 50-70°C.

Les exemples suivants illustrent l'invention sans en limiter la portée.

## EXEMPLE 1

Dans un autoclave de type Grignard en acier inoxydable d'un volume de 250 ml, muni d'une double enveloppe permettant de réguler la température à 18°C par circulation d'eau, on introduit dans l'ordre, sous atmosphère d'éthylène: 2,5 ml d'une solution de triéthylaluminium dans une coupe d'hexènes préparée en mélangeant 0,25 ml de triéthylaluminium avec 9,75 ml d'hexènes, puis une solution d'un complexe titanate de tétra-n-butyle-tétrahydrofuranne préparée en mélangeant 0,05 ml de titanate de tétra-n butyle avec 0,024 ml de tétrahydrofuranne et 2,42 ml de coupe hexènes. Le rapport molaire entre le tétrahydrofuranne et le titanate est égal à 2,1: 1. Après 2 minutes d'interaction, la température est portée à 55°C et la pression d'éthylène à 2 MPa.

Après 2h 30 de réaction, l'introduction d'éthylène est arrêtée et le catalyseur est détruit par injection sous pression de 2 ml d'eau. On a consommé au total 133 g d'éthylène.

Outre l'éthylène qui n'a pas réagi, on recueille: 0,28 g de n-butane, 92,40 g de butène-1, 6,46 g d'hexènes, 1,17g d'octènes et 0,0027 g de polyéthylène. L'analyse de la fraction $C_4$ par chromatographie en phase gazeuse avec un détecteur à ionisation de flamme montre une teneur en butène-2 total inférieure à 10 ppm. Le polyéthylène représente 27 ppm.

Dans ces conditions, la productivité du catalyseur s'élève à 13088 g de butène-1 par g de titane métal.

## EXEMPLE 2

(comparaison - ne fait pas partie de l'invention)

Dans le même appareillage que celui qui a été utilisé dans l'exemple 1 et toutes choses égales par ailleurs, on a augmenté la quantité de tétrahydrofuranne introduite de façon à avoir un rapport molaire tétrahydrofuranne sur titanate de 20: l'au lieu de 2,1: 1. On n'a constaté aucune conversion de l'éthylène après 5h à 55°C.

## EXEMPLE 3

(ne fait pas partie de l'invention)

Dans le même appareillage que celui qui a été utilisé dans l'exemple 1 et dans les mêmes conditions à ceci près qu'on a introduit 2 fois plus du composé du titane, deux fois plus du composé de l'aluminium et pas de tétrahydrofuranne, on a obtenu 91,6 g de butène-1, 0,28 g de butane, 11,8 g d'hexènes, 0,68 g d'octènes et 0,1 g de polyéthylène. Le butène-1 contient 360 ppm de butène-2. La productivité du catalyseur n'est que de 6500g de butène par g de Ti métal.

## EXEMPLE 4

(ne fait pas partie de l'invention)

Dans le même appareillage que celui qui a été utilisé dans l'exemple 1, on introduit dans l'ordre, sous atmosphère d'éthylène: 2,5 ml d'une solution de triéthylaluminium dans une coupe d'hexènes préparée en mélangeant 0,25 ml de triéthylaluminium avec 9,75 ml d'hexènes, puis 0,024 ml de tétrahydrofuranne, et enfin une solution de 0,05 ml de titanate de tétra-n-butyle dans 2,42 ml de coupe hexènes.

La réaction est ensuite mise en oeuvre dans les mêmes conditions et suivant le même protocole que décrit dans l'exemple 1.

La productivité du catalyseur n'est que de 5592 g de butène-1 par g de titane métal. Le polyéthylène représente 70 ppm.

## EXEMPLE 5

(ne fait pas partie de l'invention)

Dans le même appareillage que celui qui a été utilisé dans l'exemple 1, on introduit dans l'ordre sous atmosphère d'éthylène: 2,5 ml d'une solution de triéthylaluminium dans une coupe d'hexènes préparée en mélangeant 0,25 ml de triéthylaluminium avec 9,75 ml d'hexènes, puis une solution de 0,05 ml de titanate de tétra-n-butyle dans 2,42 ml de coupe hexènes, et enfin 0,024 ml de tétrahydrofuranne.

La réaction est ensuite mise en oeuvre dans les mêmes conditions et suivant le même protocole que décrit dans l'exemple 1.

La productivité du catalyseur est seulement de 9231 g de butène-1 par g de titane métal. Le polyéthylène représente 42 ppm.

## EXEMPLE 6

Dans le même appareillage que dans l'exemple 1, on introduit sous atmosphère d'éthylène, et en maintenant la température à 18°C: 2,5 ml d'une solution de triéthylaliminium dans une coupe d'exènes, préparée en mélangeant 0,25 ml de triéthylaluminium avec 9,75 ml d'hexènes, puis une solution d'un complexe titanate de tétra-n-butyle-dioxanne-1,4, préparée en mélangeant 0,05 ml de titanate de tétra-n-butyle avec 0,025 ml de dioxanne-1,4 et 2,42 ml de coupe hexènes. Le rapport molaire du dioxanne-1,4 au titanate est de 2,07: 1. Après 2 minutes d'interaction la température est portée à 55°C et la pression d'éthylène à 2 MPa.

Après 2h 30 de réaction, la réaction est arrêtée par introduction sous pression de 2 ml d'eau. On a consommé au total 120g d'éthylène.

Outre l'éthylène qui n'a pas réagi, on recueille: 0,18 g de n-butane, 87,09 g de butène-1, 6,88 g d'hexènes, 0,20 g d'octènes et 0,0042 g de polyéthylène. La fraction $C_4$ contient 2060 ppm de butane et un taux de butène-2 total inférieur à 10 ppm. Le polyéthylène représente 45 ppm.

La productivité du catalyseur s'élève à 12336 g de butène-1 par gramme de titane métal.

## EXEMPLE 7

Dans le même appareillage que dans l'exemple 1, on introduit sous atmosphère d'éthylène, et en maintenant la température à 18°C: 1,25 ml d'une solution de triéthylaluminium dans une coupe d'hexènes, préparée en mélangeant 0,5 ml de triéthylaluminium avec 9,5 ml d'hexènes, puis une solution d'un complexe titanate de n-butyle-glyme (éther diméthylique de l'éthylèneglycol), préparée en mélangeant 0,05 ml de titanate de n-butyle avec 0,05 ml de glyme et 2,5 ml de coupe hexènes. Le rapport molaire du glyme au titanate est de 3,2: 1.

Après 2 minutes d'interaction, la température est portée à 55°C et la pression d'éthylène à 2 MPa.

Après 2 heures de réaction, le catalyseur est détruit par addition sous pression de 2 ml d'eau. On a consommé au total 136 g d éthylène.

Outre l'éthylène qui n'a pas réagi, on recueille: 0,22 g de n-butane, 89,28 g de butène-1, 9,83 g d'hexènes, 0,41 g d'octènes et 0,0139 g de polyéthylène. La fraction $C_4$ contient 2400 ppm de n-butane et un taux de butène-2 total inférieur à 10 ppm. Le polyéthylène représente 139 ppm.

La productivité du catalyseur s'élève à 12646 g de butène-1 par gramme de titane métal.

## EXEMPLE 8

(comparaison, ne fait pas partie de l'invention)

Dans le même appareillage que dans l'exemple 5 et toutes choses égales par ailleurs, on a augmenté la quantité de glyme introduite de façon à avoir un rapport molaire glyme sur titanate de 230: 1 au lieu de 3,2: 1.

La productivité du catalyseur est alors seulement de 1350g de butène-1 par gramme de titane métal.

## EXEMPLE 9

Dans le même appareillage que dans l'exemple 1, on introduit sous atmosphère d'éthylène et en maintenant la température à 18°C: 2,5 ml d'une solution de triéthylaluminium dans une coupe d'hexènes, préparée en mélangeant 0,5 ml de triéthylaluminium avec 19,5 ml d'hexènes, puis 2,5 ml d'une solution d'un complexe titanate de n-butyle-dihydropyranne, préparée en mélangeant 0,4 ml de titanate de n-butyle avec 0,215 ml de dihydropyranne dans 19,6 ml d'hexènes. Le rapport molaire du dihydropyranne au titanate est de 2: 1.

Après 2 minutes d'interaction, la température est portée à 55°C et la pression d'éthylène à 2 MPa.

Le catalyseur est détruit après 1h 30 de réaction.

Les produits recueillis correspondent à une productivité du catalyseur de 6146 g de butène-1 par gramme de titane métal.

## EXEMPLE 10

Dans le même appareillage que dans l'exemple 1 on introduit sous atmosphère d'éthylène et en maintenant la température à 35°C: 2,5 ml d'une solution de triéthylaluminium dans l'heptane préparée en mélangeant 0,5 ml de triéthylaluminium avec 19,5 ml d'heptane, puis 2,5 ml d'une solution d'un complexe titanate d'isopropyle-dioxanne-1,4, préparée en mélangeant 0,34 ml de titanate d'isopropyle avec 0,2 ml de dioxanne-1,4 dans 19,4 ml d'heptane. Le rapport molaire du dioxanne au titanate est de 2 : 1.

Après 5 minutes d'interaction, la température est portée à 70°C et la pression d'éthylène à 3 MPa. Le catalyseur est détruit après 2 heures de réaction.

La productivité du catalyseur est de 8500 grammes de butène-1 par gramme de titane métal.

## Revendications

1. Procédé amélioré de conversion de l'éthylène en butène-1, dans lequel on met l'éthylène en contact avec un catalyseur obtenu par interaction d'un titanate d'alkyle avec un composé d'alkylaluminium, caractérisé en ce que ledit catalyseur résulte de l'interaction d'un mélange préformé de titanate d'alkyle et d'éther dans un rapport molaire éther/titanate de 0,5 : 1 à 10 : 1 avec un composé d'aluminium de formule AlR$_3$ ou AlR$_2$H dans laquelle chacun des restes R est un radical hydrocarbyle.

2. Procédé selon la revendication 1 dans lequel le rapport molaire de l'éther au titanate d'alkyle est d'environ 1 : 1 à 3 : 1.

3. Procédé selon la revendication 1 ou 2, dans lequel ladite interaction est effectuée dans un solvant constitué par les hexènes recueillis comme sous-produits de la réaction.

4. Procédé selon l'une des revendications 1 à 3 dans lequel l'éther est le diéthyléther, le diisopropyléther, le dibutyléther, le méthyltertiobutyléther, le tétrahydrofuranne, le dioxanne-1,4, le dihydropyranne ou l'éther diméthylique de l'éthylèneglycol.

5. Procédé selon la revendication 4 dans lequel l'éther est le dioxanne-1,4.

6. Procédé selon la revendication 4, dans lequel l'éther est le tétrahydrofuranne.

7. Procédé selon l'une des revendications 1 à 6, dans lequel l'interaction du mélange préformé de titanate d'alkyle et d'éther avec le composé d'aluminium est effectuée à une température de -10 à ì 45°C en atmosphère d'éthylène et la température est ensuite portée à 50-70°C dans le but de convertir l'éthylène en butène-1.

8. Procédé selon la revendication 7, dans lequel on maintient les composants mélangés du catalyseur à une température de -10 à 45°C pendant 30 secondes à 24 heures avant de porter ensuite la température jusqu'à 50-70°C.

9. Procédé selon l'une des revendications 1 à 8 dans lequel le rapport molaire entre le composé de l'aluminium et le composé de titane est de 1 : 1 à 20 : 1, et la concentration du titane est comprise entre 10$^{-4}$ et 0,5 mole par litre lors de la mise en oeuvre du catalyseur pour convertir l'éthylène en butène-1 à une température de 20 à 150°C sous une pression de 0,5 à 8 MPa.

10. Procédé selon la revendication 9 dans lequel la température est de 20 à 70°C.

## Patentansprüche

1. Verbessertes Verfahren zur Umwandlung von Äthylen in Buten-1, bei dem Äthylen mit einem Katalysator kontaktiert wird, der durch Umsetzung eines Alkyltitanats mit einer Alkylaluminiumverbindung erhalten wurde, dadurch <u>gekennzeichnet</u>, daß man einen Katalysator einsetzt, der aus der Umsetzung eines vorgeformten Gemisches aus Alkyltitanat und Ä-ther im molaren Verhältnis Äther/Titanat von 0,5 : 1 bis 10 : 1 mit einer Aluminiumverbindung der Formel AlR$_3$ oder AlR$_2$H, worin jeder der Reste R einen Kohlenwasserstoffrest bedeutet, stammt.

2. Verfahren nach Anspruch 1, dadurch <u>gekennzeichnet</u>, daß das molare Verhältnis von Äther zu Alkyltitanat etwa 1 : 1 bis 3 : 1 beträgt.

3. Verfahren nach Anspruch 1 oder 2, dadurch <u>gekennzeichnet</u>, daß die Umsetzung in einem Lösungsmittel bestehend aus den Hexenen, die als Nebenprodukte der Reaktion gewonnen werden, durchgeführt wird.

4. Verfahren nach einem der Ansprüche 1 bis 3, dadurch <u>gekennzeichnet</u>, daß als Äther Diäthyläther, Diisopropyläther, Dibutyläther, Methyltert-butyläther, Tetrahydrofuran, Dioxan-1,4, Dihydropyran oder der Dimethyläther von Ä-thylenglykol dient.

5. Verfahren nach Anspruch 4, dadurch <u>gekennzeichnet</u>, daß als Äther Dioxan-1,4 eingesetzt wird.

6. Verfahren nach Anspruch 4, dadurch <u>gekennzeichnet</u>, daß als Äther Tetrahydrofuran eingesetzt wird.

7. Verfahren nach einem der Ansprüche 1 bis 6, dadurch <u>gekennzeichnet</u>, daß die Umsetzung des vorgeformten Gemisches aus Alkyltitanat und Äther mit der Aluminiumverbindung bei einer Temperatur von -10 bis +45°C in Äthylenatmosphäre durchgeführt und die Temperatur sodann auf 50 bis 70° zur Umwandlung von Äthylen in Buten-1 gebracht wird.

8. Verfahren nach Anspruch 7, dadurch <u>gekennzeichnet</u>, daß die Gemischkomponenten

des Katalysators bei einer Temperatur von -10 bis +45° C während 30 s bis 24 h gehalten werden, bevor sodann die Temperatur auf 50 bis 70° C gebracht wird.

9. Verfahren nach einem der Ansprüche 1 bis 8, dadurch gekennzeichnet, daß das molare Verhältnis zwischen der Aluminiumverbindung und der Titanverbindung 1: 1 bis 20: 1 beträgt und die Konzentration an Titan zwischen 10 und 0,5 mol pro liter liegt zum Zeitpunkt des Einsatzes des Katalysators zur Umwandlung des Äthylens in Buten-1 bei einer Temperatur von 20 bis 150° C unter einem Druck von 0,5 bis 8 MPa.

10. Verfahren nach Anspruch 9, dadurch gekennzeichnet, daß die Temperatur 20 bis 70° C beträgt.

**Claims**

1 - An improved process for converting ethylene to 1-butene, wherein ethylene is contected with a catalyst obtained by reacting an alkyl titanate with an elkylaluminum compound, characterized in that said catalyst results from reacting a preformed mixture of alkyl titanate and ether, in a molar ratio ether/titanate from 0.5: 1 to 10: 1, with an aluminum compound of formula $AlR_3$ or $AlR_2H$, wherein each R is a hydrocarbyl radical.

2 - A process according to claim 1, wherein the molar ratio of. the ether to the alkyl titanate is from about 1: 1 to 3: 1.

3 - A process according to claim 1 or 2, wherein said reaction is performed in a solvent consisting of hexenes recovered as by-products of the reaction.

4 - A process according to one of claims 1 to 3, wherein the ether is diethylether, of isopropylether, dibutylether, methyltert-butylether, tetrahydrofuran, 1,4-dioxane, dihydropyran or ethylencglycol dimethylether.

5 - A process according to Claim 4, wherein the ether is 1,4-dioxane.

6 - A process according to claim 4, wherein the ether is tetrahydrofuran.

7 - A process according to one of claims 1 to 6, wherein the reaction of the preformed mixture of alkyl titanate and ether with the aluminum compound is performed at a temperature from -10 to +45° C in ethylene atmosphere and the temperature is then increased to 50 - 70° C to convert ethylene to 1-butene.

8 - A process according to claim 7, wherein the admixed catalyst components are maintained at a temperature from -10° to 45° C for 30 seconds to 24 hours before increasing the temperature to 50 - 70° C.

9 - A process according to one of claims 1 to 8, wherein the molar ratio of the aluminum compound to the titanium compound is from 1: 1 to 20: 1 and the titanium concentration is from 10-4 to 0.5 mule per liter when the catalyst is used for converting ethylene to 1-butene at a temperature from 20 to 150° C under a pressure from 0.5 to 8 MPa.

10 - A process according to claim 9 wherein the temperature is from 20 to 70° C.